# EUROPEAN PATENT APPLICATION

(11) **EP 4 438 051 A1**
(43) Date of publication of application: **02.10.2024**
(21) Application number: 24159416.7
(22) Date of filing: 23.02.2024
(51) Int. Cl.: A61K 35/741, A61K 35/745, A61P 31/04, A23L 33/135, A23K 10/18

(54) **MICROBIOME-BASED COMPOSITION FOR PREVENTING AND TREATING MULTIDRUG RESISTANT BACTERIAL INFECTIONS**

(30) Priority: 28.03.2023 KR 20230040382; 28.06.2023 KR 20230083791; 19.02.2024 KR 20240023521
(71) Applicant: Biome Inc., Seodaemun-gu Seoul 03722 (KR); Industry-Academic Cooperation Foundation, Yonsei University, Seoul 03722 (KR)
(72) Inventor: YOON, Sang Sun, Seoul (KR); CHOI, Jun Yong, Seoul (KR); YOON, Mi Young, Seoul (KR); JEONG, Uk Jin, Seoul (KR)
(74) Representative: Fabry, Bernd

(57) **Abstract**

The present invention provides an antibacterial composition including two or more strains selected from the group consisting of *Anaerostipes hadrus*, *Collinsella aerofaciens*, *Blautia wexlerae* and *Bifidobacterium longum* strains that inhibit the activity of antibiotic-resistant bacteria.

## Description

### [Technical Field]

The present invention relates to a microbiome-based composition for preventing and treating multidrug-resistant bacterial infections.

### [Background Art]

Vancomycin-resistant *Enterococci* (VRE), one of multidrug-resistant bacteria, is a bacterium that has a round egg-like shape and mainly infects the gastrointestinal tract and the genitourinary system. The VRE may cause infections such as urinary tract infections, wound infections, bacteremia, and endocarditis in the elderly, immunocompromised patients, patients with chronic underlying diseases, or patients in hospitals.

Furthermore, the VRE is one of bacteria that are difficult to be treated due to resistance not only to vancomycin, which is known to be a broad-spectrum antibiotic, but also to penicillin and cephalosporin antibiotics. Patients infected with the VRE may experience serious health problems if the patients do not receive antibiotic treatment. In particular, when bacteremia is caused by the VRE, the mortality is reported to be 67%, which is 2 to 3 times higher than that of a control group. However, since VRE infection generally occurs in patients with poor underlying diseases, it is presumed that these underlying diseases contribute to increasing mortality.

To date, a treatment method for patients showing VRE infection symptoms is mainly performed by selecting and treating antibiotics that are non-resistant and susceptible according to an antibiotic susceptibility test result, but it may be difficult to be treated due to an increase of VRE strains according to widespread use of antibiotics.

In addition, carbapenem is one of important antibiotics against bacteria resistant to cephalosporins or broad-spectrum *β*-lactamase. Carbapenem-resistant *Enterobacteriaceae* (CRE) refers to a bacterium that has acquired resistance to carbapenem-based antibiotics, such as *Escherichia coli, Klebsiella pneumoniae,* and *Enterobacter cloacae,* which cause various infections such as urinary tract infections, pneumonia, and sepsis. Patients with CRE infections are often resistant to several classes of antibiotics to be hardly treated and may cause a variety of serious infections such as bloodstream infections, pneumonia, and urethral infections due to limited antibiotics that may be used for treatment.

Microbiome refers to the community of microbial strains that play beneficial roles in the human body. According to recent studies, there has been made the possibility that the microbiome could also be used to prevent or treat pathogen infections. In fact, there is a case where microbiome treatment is shown to be effective in *Clostridium difficile* infection, one of causative bacteria that cause dangerous diarrhea and colitis by disrupting the intestinal bacterial balance.

Accordingly, there is a greater need for the development of an antibacterial composition, a pharmaceutical composition, and a method using the same that can inhibit antibiotic-resistant bacteria, including VRE and CRE infections, and prevent antibiotic-resistant bacterial infections by improving intestinal imbalance caused by VRE and CRE antibiotic-resistant bacteria.

The background art of the invention has been prepared to facilitate easier understanding of the present invention. It should not be understood that the matters described in the background art of the invention exist as prior arts.

### [Disclosure]

### [Technical Problem]

Meanwhile, the present inventors recognized the need for microbial strains that are habitable in the gut and inhibit the activity of antibiotic-resistant bacteria.

Furthermore, it was noted that the ability of inhibiting antibiotic-resistant bacteria was doubled when treated in combination with microbial strains that inhibited the activity of antibiotic-resistant bacteria, compared to when treated with the microbial strain alone that inhibited the activity of antibiotic-resistant bacteria.

Accordingly, the present inventors intended to develop an antibacterial composition consisting of a combination of microbial strains that are inhabitable in the gut and are excellent in inhibiting antibiotic-resistant bacteria.

As a result, the present inventors found that an antibacterial composition including two or more strains selected from the group consisting of *Anaerostipes hadrus, Collinsella aerofaciens, Blautia wexlerae,* and *Bifidobacterium longum* of the present invention was excellent in inhibiting the activity of antibiotic-resistant bacteria.

Therefore, an object of the present invention is to provide an antibacterial composition including two or more strains selected from the group consisting of *Anaerostipes hadrus, Collinsella aerofaciens, Blautia wexlerae,* and *Bifidobacterium longum.*

Another object of the present invention is to provide a pharmaceutical composition, a health functional food, and a feed additive for preventing or treating antibiotic-resistant bacterial infections, including as an active ingredient at least one selected from the group consisting of two or more strains selected from the group consisting of *Anaerostipes hadrus, Collinsella aerofaciens, Blautia wexlerae,* and *Bifidobacterium longum,* a culture of the strain, a concentrate of the culture, a dried product of the culture, and combinations thereof. Yet another object of the present invention is to provide a method for treating antibiotic-resistant bacterial infections in animals other than humans.

The objects of the present invention are not limited to the aforementioned objects, and other objects, which are not mentioned above, will be apparent to those skilled in the art from the following description.

### [Technical Solution]

In order to solve the problems described above, one aspect of the present invention provides an antibacterial composition including two or more strains selected from the group consisting of *Anaerostipes hadrus, Collinsella aerofaciens, Blautia wexlerae* and *Bifidobacterium longum* strains that inhibit the activity of antibiotic-resistant bacteria according to an embodiment of the present invention.

According to a feature of the present invention, the *Anaerostipes hadrus, Collinsella aerofaciens, Blautia wexlerae* and *Bifidobacterium longum* strains may be isolated from human feces.

According to another feature of the present invention, the *Anaerostipes hadrus* may be *Anaerostipes hadrus* (RC1, accession number: KCTC15409BP), the *Collinsella aerofaciens* may be *Collinsella aerofaciens* (RC2, accession number: KCTC15408BP), the *Blautia wexlerae* may be *Blautia wexlerae* (RC3, accession number: KCTC15396BP), and the *Bifidobacterium longum* may be *Bifidobacterium longum* (RC4, accession number: KCTC15416BP).

According to another feature of the present invention, the composition of the present invention may include *Anaerostipes hadrus* (accession number: KCTC15409BP), *Collinsella aerofaciens* (accession number: KCTC15408BP), and *Blautia wexlerae* (accession number: KCTC15396BP), but is not limited thereto.

According to another feature of the present invention, the composition of the present invention may include *Anaerostipes hadrus* (accession number: KCTC15409BP), *Blautia wexlerae* (accession number: KCTC15396BP), and *Bifidobacterium longum* (accession number: KCTC15416BP), but is not limited thereto.

According to another feature of the present invention, the composition of the present invention may include *Collinsella aerofaciens* (accession number: KCTC15408BP), *Blautia wexlerae* (accession number: KCTC15396BP), and *Bifidobacterium longum* (accession number: KCTC15416BP), but is not limited thereto.

According to yet another feature of the present invention, the strains may have antibiotic-resistant bacteria inhibitory activity, and the aforementioned antibiotic-resistant bacteria may be at least one antibiotic-resistant bacteria selected from the group consisting of multidrug-resistant bacteria, including Vancomycin-resistant *Enterococci* (VRE) or Carbapenem-resistant *Enterobacteriaceae* (CRE), *Methicillin-resistant Staphylococcus aureus* (MRSA), *Streptococcus pyogenes* (Group A Streptococcus, GAS), acute *Leptospira spp, Streptococcus pneumoniae, Micrococcus luteus, Bacillus subtilis, Listeria monocytogenes, Corynebacterium diphtheriae, Francisella spp., Nocardia spp.,* and *Corynebacterium jeikeium,* but are not limited thereto.

According to yet another feature of the present invention, the *Anaerostipes hadrus* may be a strain identified by a base sequence having at least 97% homology with a 16S rDNA base sequence represented by SEQ ID NO: 1, the *Collinsella aerofaciens* may be a strain identified by a base sequence having at least 97% homology with a 16S rDNA base sequence represented by SEQ ID NO: 2, the *Blautia wexlerae* may be a strain identified by a base sequence having at least 97% homology with a 16S rDNA base sequence represented by SEQ ID NO: 3, and the *Bifidobacterium longum* may be a strain identified by a base sequence having at least 97% homology with a 16S rDNA base sequence represented by SEQ ID NO: 4.

At this time, the SEQ ID NOs: 1 to 4 may be as follows.
SEQ ID NO: 1: 
GGAGGGGCGGCGTGCTTACACGTGGGAGTCTGACGAAACACCTTA
TTTGATTTTCTTCGGAACTGAAGATTTGGTGATTGAGTGGCGGACGGGTG
AGTAACGCGTGGGTAACCTGCCCTGTACAGGGGGATAACAGTCAGAAAT
GACTGCTAATACCGCATAAGACCACAGCACCGCATGGTGCAGGGGTAAA
AACTCCGGTGGTACAGGATGGACCCGCGTCTGATTAGCTGGTTGGTGAGG
TAACGGCTCACCAAGGCGACGATCAGTAGCCGGCTTGAGAGAGTGAACG
GCCACATTGGGACTGAGACACGGCCCAAACTCCTACGGGAGGCAGCAGT
GGGGAATATTGCACAATGGGGGAAACCCTGATGCAGCGACGCCGCGTGA
GTGAAGAAGTATCTCGGTATGTAAAGCTCTATCAGCAGGGAAGAAAATG
ACGGTACCTGACTAAGAAGCCCCGGCTAACTACGTGCCAGCAGCCGCGG
TAATACGTAGGGGGCAAGCGTTATCCGGAATTACTGGGTGTAAAGGGTG
CGTAGGTGGTATGGCAAGTCAGAAGTGAAAACCCAGGGCTTAACTCTGG
GACTGCTTTTGAAACTGTCAGACTGGAGTGCAGGAGAGGTAAGCGGAAT
TCCTAGTGTAGCGGTGAAATGCGTAGATATTAGGAGGAACATCAGTGGC
 
GAAGGCGGCTTACTGGACTGAAACTGACACTGAGGCACGAAAGCGTGGG
GAGCAAACAGGATTAGATACCCTGGTAGTCCACGCCGTAAACGATGAAT
ACTAGGTGTCGGGGCCGTAGAGGCTTCGGTGCCGCAGCCAACGCAGTAA
GTATTCCACCTGGGGAGTACGTTCGCAAGAATGAAACTCAAAGGAATTG
ACGGGGACCCGCACAAGCGGTGGAGCATGTGGTTTAATTCGAAGCAACG
CGAAGAACCTTACCTGGTCTTGACATCCTTCTGACCGGTCCTTAACCGGA
CCTTTCCTTCGGGACAGGAGTGACAGGTGGTGCATGGTTGTCGTCAGCTC
GTGTCGTGAGATGTTGGGTTAAGTCCCGCAACGAGCGCAACCCCTATCTT
TAGTAGCCAGCAATATAAGGTGGGCACTCTAGAGAGACTGCCAGGGATA
ACCTGGGAGGAAGGTGGGGACGACGTCAAATCATCATGCCCCTTATGAC
CAGGGCTACCCCCGTGCTACAATGGGCTTAAACCAAGGGGAAA
SEQ ID NO: 2: 
GGAATTGGCGGGCCGCTACACATGCAGTCGAACGGCACCCCTCTC
CGGAGGGAAGCGAGTGGCGAACGGCTGAGTAACACGTGGAGAACCTGCC
CCCTCCCCCGGGATAGCCGCCCGAAAGGACGGGTAATACCGGATACCCC
CGGGCGCCGCATGGCGCCCGGGCTAAAGCCCCGACGGGAGGGGATGGCT
CCGCGGCCCATCAGGTAGACGGCGGGGTGACGGCCCACCGTGCCGACAA
CGGGTAGCCGGGTTGAGAGACCGACCGGCCAGATTGGGACTGAGACACG
GCCCAGACTCCTACGGGAGGCAGCAGTGGGGAATCTTGCGCAATGGGGG
GAACCCTGACGCAGCGACGCCGCGTGCGGGACGGAGGCCTTCGGGTCGT
AAACCGCTTTCAGCAGGGAAGAGTCAAGACTGTACCTGCAGAAGAAGCC
CCGGCTAACTACGTGCCAGCAGCCGCGGTAATACGTAGGGGGCGAGCGT
TATCCGGATTCATTGGGCGTAAAGCGCGCGTAGGCGGCCCGGCAGGCCG
GGGGTCGAAGCGGGGGGCTCAACCCCCCGAAGCCCCCGGAACCTCCGCG
GCTTGGGTCCGGTAGGGGAGGGTGGAACACCCGGTGTAGCGGTGGAATG
 
CGCAGATATCGGGTGGAACACCGGTGGCGAAGGCGGCCCTCTGGGCCGA
GACCGACGCTGAGGCGCGAAAGCTGGGGGAGCGAACAGGATTAGATACC
CTGGTAGTCCCAGCCGTAAACGATGGACGCTAGGTGTGGGGGGACGATC
CCCCCGTGCCGCAGCCAACGCATTAAGCGTCCCGCCTGGGGAGTACGGC
CGCAAGGCTAAAACTCAAAGGAATTGACGGGGGCCCGCACAAGCAGCGG
AGCATGTGGCTTAATTCGAAGCAACGCGAAGAACCTTACCAGGGCTTGA
CATATGGGTGAAGCGGGGGAGACCCCGTGGCCGAGAGGAGCCCATACAG
GTGGTGCATGGCTGTCGTCAGCTCGTGTCGTGAGATGTTGGGTTAAGTCC
CGCAACGAGCGCAACCCCCGCCGCGTGTTGCCATCGGGTGATGCCGGGA
ACCCACGCGGGACCGCCGCCGTCAAGGCGGAAGGAGGGCGGGGACGAC
GTCAAGTCATCATGCCCCTTATGCCCTGGGCTGCACACGTGCTACAATGG
GCCGGTACAAAGGGATGCCCCCCCCGCGAGGGGGAACCGGATCCCGGAA
AGCCGGCCCC
SEQ ID NO: 3: 
TGAATGGCCGCGTGCTTACACATGCAGTCGAACGGGAATATATCT

TCTATTGAGACTTCGGTGGATTTAGATTCTAATTTCTAGTGGCGGACGGG

TGAGTAACGCGTGGGTAACCTGCCTTATACAGGGGGATAACAGTCAGAA

ATGGCTGCTAATACCGCATAAGCGCACAGAGCTGCATGGCTCAGTGTGA

AAAACTCCGGTGGTATAAGATGGACCCGCGTTGGATTAGCTTGTTGGTGG

GGTAACGGCCCACCAAGGCGACGATCCATAGCCGGCCTGAGAGGGTGAA

CGGCCACATTGGGACTGAGACACGGCCCAGACTCCTACGGGAGGCAGCA

GTGGGGAATATTGCACAATGGGGGAAACCCTGATGCAGCGACGCCGCGT

GAAGGAAGAAGTATCTCGGTATGTAAACTTCTATCAGCAGGGAAGATAG

TGACGGTACCTGACTAAGAAGCCCCGGCTAACTACGTGCCAGCAGCCGC

GGTAATACGTAGGGGGCAAGCGTTATCCGGATTTACTGGGTGTAAAGGG
 
AGCGTAGACGGTGTGGCAAGTCTGATGTGAAAGGCATGGGCTCAACCTG
TGGACTGCATTGGAAACTGTCATACTTGAGTGCCGGAGGGGTAAGCGGA
ATTCCTAGTGTAGCGGTGAAATGCGTAGATATTAGGAGGAACACCAGTG
GCGAAGGCGGCTTACTGGACGGTAACTGACGTTGAGGCTCGAAAGCGTG
GGGAGCAAACAGGATTAGATACCCTGGTAGTCCACGCCGTAAACGATGA
ATACTAGGTGTCGGGGAGCATAGCTCTTCGGTGCCGTCGCAAACGCAGTA
AGTATTCCACCTGGGGAGTACGTTCGCAAGAATGAAACTCAAAGGAATT
GACGGGGACCCGCACAAGCGGTGGAGCATGTGGTTTAATTCGAAGCAAC
GCGAAGAACCTTACCAAGTCTTGACATCCGTCTGACCGATCCTTAATCGG
ATCTTTCCTTCGGGACAGGCGAGACAGGTGGTGCATGGTTGTCGTCAGCT
CGTGTCGTGAGATGTTGGGTTAAGTCCCGCAACGAGCGCAACCCCTATCC
TCAGTAGCCAGCATTTAAGGTGGGCACTCTGGGGAGACTGCCAGGGATA
ACCTGGAGGAAGGCGGGGATGACGTCAAATCATCATGCCCCTTATGATTT
GGGCTACCACCTGCTACAATGGGGTAACCAAGGGAGACGGAAATGGTGG
AAAGGGGGCAAT
SEQ ID NO: 4:

GGGAATTTGCGGCGTGCTTACACATGCAAGTCGAACGGGATCCAT
CAGGCTTTGCTTGGTGGTGAGAGTGGCGAACGGGTGAGTAATGCGTGAC
CGACCTGCCCCATACACCGGAATAGCTCCTGGAAACGGGTGGTAATGCC
GGATGCTCCAGTTGATCGCATGGTCTTCTGGGAAAGCTTTCGCGGTATGG
GATGGGGTCGCGTCCTATCAGCTTGACGGCGGGGTAACGGCCCACCGTG
GCTTCGACGGGTAGCCGGCCTGAGAGGGCGACCGGCCACATTGGGACTG
AGATACGGCCCAGACTCCTACGGGAGGCAGCAGTGGGGAATATTGCACA
ATGGGCGCAAGCCTGATGCAGCGACGCCGCGTGAGGGATGGAGGCCTTC
GGGTTGTAAACCTCTTTTATCGGGGAGCAAGCGAGAGTGAGTTTACCCGT

TGAATAAGCACCGGCTAACTACGTGCCAGCAGCCGCGGTAATACGTAGG
GTGCAAGCGTTATCCGGAATTATTGGGCGTAAAGGGCTCGTAGGCGGTTC
GTCGCGTCCGGTGTGAAAGTCCATCGCTTAACGGTGGATCCGCGCCGGGT
ACGGGCGGGCTTGAGTGCGGTAGGGGAGACTGGAATTCCCGGTGTAACG
GTGGAATGTGTAGATATCGGGAAGAACACCAATGGCGAAGGCAGGTCTC
TGGGCCGTTACTGACGCTGAGGAGCGAAAGCGTGGGGAGCGAACAGGAT
TAGATACCCTGGTAGTCCACGCCGTAAACGGTGGATGCTGGATGTGGGG
CCCGTTCCACGGGTTCCGTGTCGGAGCTAACGCGTTAAGCATCCCGCCTG
GGGAGTACGGCCGCAAGGCTAAAACTCAAAGAAATTGACGGGGGCCCGC
ACAAGCGGCGGAGCATGCGGATTAATTCGATGCAACGCGAAGAACCTTA
CCTGGGCTTGACATGTTCCCGACGGTCGTAGAGATACGGCTTCCCTTCGG
GGCGGGTTCACAGGTGGTGCATGGTCGTCGTCAGCTCGTGTCGTGAGATG
TTGGGTTAAGTCCCGCAACGAGCGCAACCCTCGCCCCGTGTTGCCAGCGG
ATTATGCCGGGAACTCACGGGGGACCGCCGGGGTAACTCGGAGGAAGGT
GGGGATGACGTCAGATCATCATGCCCCTTACGTCCAGGGCTTCCGCATGC
TACAATGGCCGGTACAACGGGATGCAACCCGGGAACCCGGAACGGATCC
CTGAAAACCGGTTT

According to yet another feature of the present invention, the *Anaerostipes hadrus* may be a strain identified by the 16S rDNA base sequence represented by SEQ ID NO: 1, the *Collinsella aerofaciens* may be a strain identified by the 16S rDNA sequence represented by SEQ ID NO: 2, the *Blautia wexlerae* may be a strain identified by the 16S rDNA sequence represented by SEQ ID NO: 3, and the *Bifidobacterium longum* may be a strain identified by the 16S rDNA sequence represented by SEQ ID NO: 4.

Another aspect of the present invention provides a pharmaceutical composition for preventing or treating vancomycin-resistant *Enterococci* (VRE) infections. At this time, the aforementioned pharmaceutical composition includes as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit the activity of vancomycin-resistant *Enterococci,* a culture of the strain, a concentrate of the culture, a dried product of the culture, and combinations thereof.

Meanwhile, the culture of the above-mentioned strains may be a sediment fraction obtained by centrifuging the strain culture, the concentrate may be concentrated by depressurizing the culture in a vacuum concentrator, and the dried product may be a dried product obtained by drying the above-mentioned concentrate in a hot air dryer.

According to a feature of the present invention, the *Anaerostipes hadrus, Collinsella aerofaciens, Blautia wexlerae,* and *Bifidobacterium longum* strains may be administered by one or more routes selected from the group consisting of oral, rectal, and colonic routes.

Yet another aspect of the present invention provides a health functional food for inhibiting the activity of antibiotic-resistant bacteria. At this time, the aforementioned health functional food includes as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit the activity of antibiotic-resistant bacteria, a culture of the strain, a concentrate of the culture, a dried product of the culture, and combinations thereof.

Yet another aspect of the present invention provides a feed additive for inhibiting the activity of antibiotic-resistant bacteria. At this time, the aforementioned feed additive includes as an active ingredient at least one selected from the group consisting of two or more strains selected from the group consisting of *Anaerostipes hadrus, Collinsella aerofaciens, Blautia wexlerae,* and *Bifidobacterium longum,* which inhibit the activity of antibiotic-resistant bacteria, a culture of the strain, a concentrate of the culture, a dried product of the culture, and combinations thereof.

Yet another aspect of the present invention provides a method for treating antibiotic-resistant bacterial infections in animals other than humans, including administering the above-described composition to a subject other than a human suspected of having antibiotic-resistant bacterial infections.

Hereinafter, the present invention will be described in more detail with reference to Examples. However, these Examples are only illustrative of the present invention, and the scope of the present invention is not limited to these Examples.

### [Advantageous Effects]

According to the present invention, it is possible to effectively inhibit or suppress the activity of antibiotic-resistant bacteria by using an antibacterial composition consisting of a combination of two or more strains selected from the group consisting of *Anaerostipes hadrus, Collinsella aerofaciens, Blautia wexlerae,* and *Bifidobacterium longum* strains.

However, a single composition of the aforementioned *Anaerostipes hadrus, Collinsella aerofaciens, Blautia wexlerae,* and *Bifidobacterium longum* strains showed lower ability of inhibiting the activity of antibiotic-resistant bacteria than that of a combined composition of two or more of the strains.

Therefore, the present invention may provide an antibacterial composition and a pharmaceutical composition that have the effect of preventing or treating infections caused by antibiotic-resistant bacteria through combinations of strains with the ability of inhibiting or suppressing antibiotic-resistant bacteria based on the microbiome.

The effects of the present invention are not limited by the foregoing, and other various effects are anticipated herein.

### [Description of Drawings]

FIG. 1 lists names of strains that inhibit the activity of antibiotic-resistant bacteria included in an antibacterial composition according to another embodiment of the present invention.
FIG. 2 shows results of inhibiting the activity of Vancomycin-resistant *Enterococci* according to combinations of strains that inhibit the activity of antibiotic-resistant bacteria according to yet another embodiment of the present invention.
FIGS. 3A to 3E show results of inhibiting the activity of Vancomycin-resistant *Enterococci* according to combinations of strains that inhibit the activity of antibiotic-resistant bacteria using a mouse infectious model according to various embodiments of the present invention.
FIG. 4 shows comparison of effects of inhibiting (suppressing) Vancomycin-resistant *Enterococci* (VRE) on combinations (combinations of antibiotic-resistant bacteria inhibitory strains, hereinafter VRE) of four strains RC1, RC2, RC3, and RC4 according to various embodiments of the present invention and Bronco Vaxom (BV), currently available as an infection prevention agent, as a comparative group.
FIGS. 5A and 5B show results of inhibiting the activity of Carbapenem-resistant *Enterobacteriaceae* (CRE) according to administration of an antibiotic-resistant bacteria inhibitory strain (hereinafter, BM111) of the present invention in a mouse infectious model according to various embodiments of the present invention.

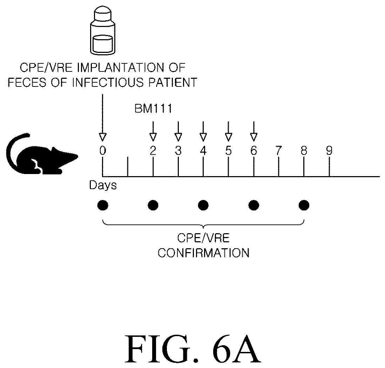

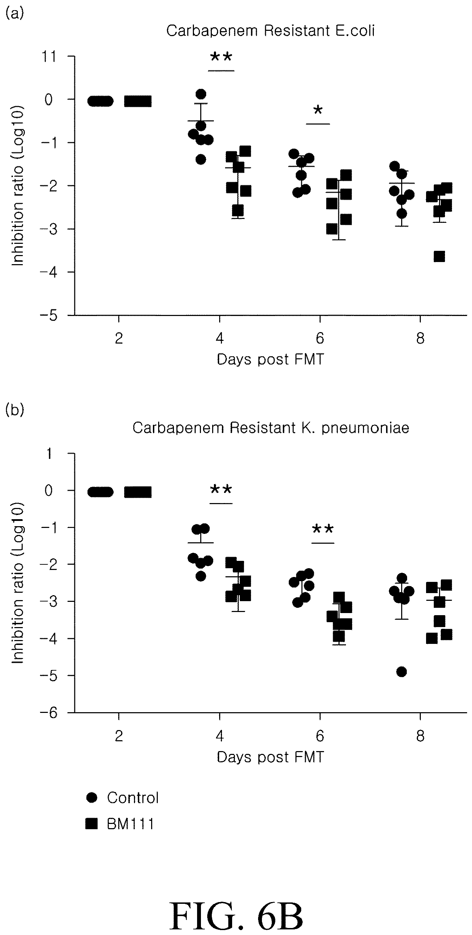

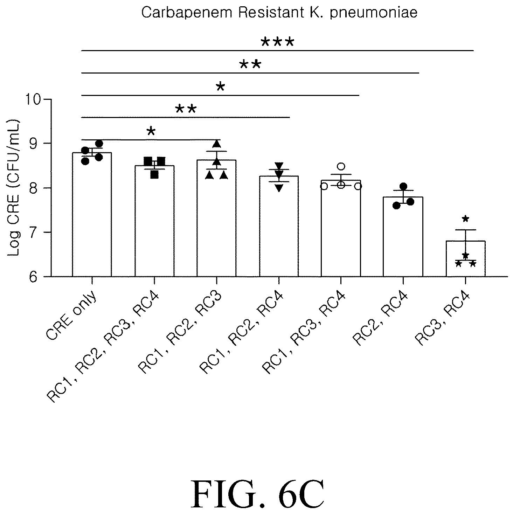

FIGS. 6A, 6B(a), and 6B(b) show results of inhibiting the activity of *Escherichia coli* and *Klebsiella pneumoniae* as Carbapenem-resistant *Enterobacteriaceae* according to administration of an antibiotic-resistant bacteria inhibitory strain (hereinafter, BM111) of the present invention in a humanized mouse infectious model according to various embodiments of the present invention. FIG. 6C shows results of inhibiting the activity of *Klebsiella pneumoniae* as Carbapenem-resistant *Enterobacteriaceae* according to a combination of strains that inhibit the activity of antibiotic-resistant bacteria according to yet another embodiment of the present invention.

### [Modes for the Invention]

Advantages and features of the present invention, and methods for accomplishing the same will be more clearly understood from embodiments to be described below in detail with reference to the accompanying drawings. However, the present invention is not limited to the embodiments set forth below and will be embodied in various different forms. The embodiments are just for rendering the disclosure of the present invention complete and are set forth to provide a complete understanding of the scope of the invention to those skilled in the art to which the present invention pertains, and the present invention will only be defined by the scope of the claims.

Hereinafter, terms used in the present specification will be described for clarity of explanation.

As used herein, the term "subject" may refer to animals, including humans, with infections caused by antibiotic-resistant bacteria, specifically, infections caused by Vancomycin-resistant *Enterococci* or Carbapenem-resistant *Enterobacteriaceae* even among multidrug-resistant bacteria, and may be used interchangeably with terms such as a host, an individual, and a patient.

As used herein, the term "multidrug-resistant bacteria" may refer to bacteria or strains of bacteria or microorganisms that are resistant to various antibiotics. These multidrug-resistant bacteria are difficult to be treated with general antibiotics, so that there is a high possibility that treatment will be delayed or failed, and thus there is a need for active prevention and treatment thereof.

As used herein, the term "antibiotic-resistant bacteria" may be at least one antibiotic-resistant bacteria selected from the group consisting of multidrug-resistant bacteria, including Vancomycin-resistant *Enterococci* (VRE) or Carbapenem-resistant *Enterobacteriaceae* (CRE), *Methicillin-resistant Staphylococcus aureus* (MRSA), *Streptococcus pyogenes* (Group A Streptococcus, GAS), acute *Leptospira spp, Streptococcus pneumoniae, Micrococcus luteus, Bacillus subtilis, Listeria monocytogenes, Corynebacterium diphtheriae, Francisella spp., Nocardia spp.,* and *Corynebacterium jeikeium,* but is not limited thereto. Furthermore, an increase in the activity of antibiotic-resistant bacteria may mean an increase in the number of antibiotic-resistant strains, and conversely, inhibition or suppression of the activity of antibiotic-resistant bacteria may mean a decrease in the number of antibiotic-resistant strains. As used herein, the term "Vancomycin-resistant *Enterococci* (VRE)" may refer to enteric bacteria that are resistant to Vancomycin (bactericidal antibiotic). Specifically, the VRE strain used in various embodiments of the present invention may be strains belonging to the *Enterococcus* genus, including one or more strains selected from the group consisting of *Enterococcus faecium, Enterococcus faecalis, Enterococcus casseliflavus, Enterococcus gallinarum, Enterococcus raffinosus, Enterococcus durans,* and *Enterococcus hirae,* but is not limited thereto, and preferably *Enterococcus faecium.* Furthermore, an increase in VRE activity may mean an increase in the number of Vancomycin-resistant *Enterococci* strains, and conversely, inhibition or suppression of VRE activity may mean a decrease in the number of *Vancomycin-resistant Enterococci* strains.

As used herein, the term "Carbapenem-resistant *Enterobacteriaceae* (CRE)" may refer to enteric bacteria that are resistant to Carbapenem. Specifically, the CRE strain used in various embodiments of the present invention may be strains belonging to the *Enterobacteriaceae* genus, including one or more strains selected from the group consisting of *Escherichia coli, Klebsiella pneumoniae,* and *Enterobacter cloacae,* but is not limited thereto, and may be preferably *Escherichia coli* and *Klebsiella pneumoniae.* Furthermore, an increase in CRE activity may mean an increase in the number of Carbapenem-resistant strains, and conversely, inhibition or suppression of CRE activity may mean a decrease in the number of Carbapenem-resistant strains.

As used herein, the term "TSMVH medium" may refer to a liquid medium prepared by adding vitamin K1, L-cysteine, hemin, and menadione to a Tryptic Soy Broth (hereinafter, referred to as TSB), a medium widely used for microbial culture.

As used herein, the term "anaerobic culture condition" may refer to a culture environment under atmospheric conditions of 95% nitrogen and 5% carbon dioxide.

As used herein, the term "synchronous culture" may refer to a culture method in which the division and growth cycles of cells or microorganisms are synchronized so that various types of cells or microorganisms divide and grow simultaneously.

As used herein, the term "gut" may refer to the inside of the digestive organs (gut) of a mammal, such as stomach, duodenum, small intestine, and large intestine. Furthermore, the gut may mean a habitat environment similar to the intestinal environment.

As used herein, the term "antibacterial composition" may mean a composition prepared for the purpose of inhibiting or killing the growth of pathogens such as bacteria, viruses, and fungi.

As used herein, the term "pharmaceutical composition" may mean a composition prepared for the purpose of preventing or treating diseases.

At this time, Vancomycin-resistant *Enterococci* or Carbapenem-resistant bacteria are described as examples of strains inhibited by the antibacterial composition and the pharmaceutical composition used in various embodiments of the present invention, but the inhibitory effect of the pharmaceutical composition used in various embodiments of the present invention is not limited to Vancomycin-resistant *Enterococci* or Carbapenem-resistant bacteria. For example, the pharmaceutical composition may be used as a pharmaceutical composition for inhibiting the activity of at least one antibiotic-resistant bacteria selected from the group consisting of multidrug-resistant bacteria, including Vancomycin-resistant *Enterococci* (VRE) or Carbapenem-resistant *Enterobacteriaceae* (CRE), *Methicillin-resistant Staphylococcus aureus* (MRSA), *Streptococcus pyogenes* (Group A Streptococcus, GAS), acute *Leptospira spp, Streptococcus pneumoniae, Micrococcus luteus, Bacillus subtilis, Listeria monocytogenes, Corynebacterium diphtheriae, Francisella spp., Nocardia spp.,* and *Corynebacterium jeikeium.*

Meanwhile, a pharmaceutically effective amount and an effective dose of the pharmaceutical composition according to various embodiments of the present invention may vary depending on a formulation method, an administration method, an administration time, and/or an administration route of the pharmaceutical composition.

First, as a formulation, the pharmaceutical composition may preferably be provided in a probiotic formulation or a lyophilisate formulation. Furthermore, for formulation as a pharmaceutical composition, the pharmaceutical composition may be further mixed with at least one diluent and excipient selected from the group consisting of lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil.

Accordingly, the pharmaceutical composition for inhibiting the activity of Vancomycin-resistant *Enterococci* according to an embodiment of the present invention may be provided even in the form of oral formulations such as powders, granules, tablets, capsules, suspensions, emulsions, syrups, and aerosols, external preparations, suppositories, and sterile injectable solutions, through the aforementioned diluents and excipients.

Furthermore, the pharmaceutical composition may further include a pharmaceutically acceptable carrier, excipient, or diluent. The pharmaceutically acceptable carrier, excipient, or diluent may include at least one selected from the group consisting of lactose, dextrose, sucrose, calcium silicate, cellulose, methylcellulose, microcrystalline cellulose, polyvinylpyrrolidone, water, methyl hydroxybenzoate, propyl hydroxybenzoate, talc, magnesium stearate, and mineral oil, but is not limited thereto.

Next, the administration route of the pharmaceutical composition may be preferably one or more routes selected from the group consisting of oral, rectal, and colonic routes. More specifically, the lyophilisate formulation may be administered through various routes after thawing according to the work guidelines for thawing provided by a supplier.

Furthermore, depending on a location of the lesion, administration may be performed by methods known in the art. For example, the pharmaceutical composition may be administered directly to a subject by any means through a route such as intravenous, intramuscular, oral, transdermal, mucosal, intranasal, intratracheal or subcutaneous administration. The pharmaceutical composition may be administered systemically or locally. Those skilled in the art may easily determine and prescribe an effective dose for a desired treatment.

Meanwhile, the pharmaceutical composition according to various embodiments of the present invention may be administered once a day or may be administered several divided times. Accordingly, the dose does not limit the scope of the present invention in any aspect.

Furthermore, the dose may vary depending on various factors including the type and degree of a response to be achieved by administration of the pharmaceutical composition, the type, age, weight, and general health condition, symptoms or severity of a disease, sex, diet, and excretion of a subject to be administered, drugs used simultaneously or separately for the corresponding subject, other composition ingredients, and the like, and similar factors well-known in the field of medicine.

As used herein, the term "active ingredient" or "effective amount" may mean any amount of the composition sufficient to alleviate, inhibit the progression of, or prevent a disease, a disorder, a condition, or one or more symptoms thereof.

As used herein, the term "prevention" refers to all actions that approach a normal control group by suppressing or delaying the symptoms of diseases caused by antibiotic-resistant bacteria by administering the strain composition or pharmaceutical composition according to various embodiments of the present invention.

As used herein, the term "treatment" refers to all actions that improve or beneficially change symptoms of a disease caused by antibiotic-resistant bacteria to be the same as or similar to a normal control group by administration of the strain composition or pharmaceutical composition according to various embodiments of the present invention.

As used herein, the term "administering" is used interchangeably with "introducing" or "implanting" and may mean disposition of a composition according to an embodiment into a subject by a method or route that causes at least partial localization of the composition according to an embodiment to a desired site. The composition or at least a portion of the composition ingredients according to an embodiment may be administered by any suitable route to be delivered to a desired location within a living subject.

As used herein, the term "health functional food" may be in the form of drinks, meat, sausage, bread, biscuits, rice cake, chocolate, candies, snacks, confectionery, pizza, ramen, other noodles, gum, dairy products including ice cream, various soups, beverages, alcoholic beverages, vitamin complex, etc., and the health functional food includes all health functional foods in the conventional sense.

Meanwhile, the active ingredients of the present invention included in the health functional food according to various embodiments of the present invention may be added to the food as it is or used together with other foods or food ingredients and may be used appropriately according to conventional methods. The mixing amount of the active ingredients may be suitably determined according to the purpose of use thereof (for prevention or alleviation). In general, the amount of the active ingredients in the health functional food may be added in an amount of 0.1 to 90 parts by weight of the total food weight. However, in the case of long-term ingestion for the purpose of health and hygiene or health regulation, the amount may be the range or less and the active ingredients may be used even in the amount of the range or more.

Furthermore, the health functional beverage composition according to various embodiments of the present invention is not particularly limited to other ingredients other than containing the active ingredient as an essential ingredient in an indicated ratio, and may contain various flavoring agents, natural carbohydrates, or the like as an additional ingredient, like conventional beverages. Examples of the above-described natural carbohydrates include general sugars, including monosaccharides, such as glucose, fructose, etc.; disaccharides, such as maltose, sucrose, etc.; and polysaccharides, such as dextrin, cyclodextrin, etc., and sugar alcohols such as xylitol, sorbitol, erythritol, etc. As flavoring agents other than those described above, natural flavoring agents (thaumatin, stevia extract (e.g., Rebaudioside A, glycyrrhizin, etc.)) and synthetic flavoring agents (saccharin, aspartame, etc.) may be used. The ratio of the natural carbohydrates may be generally about 1 to 20 g, or about 5 to 12 g per 100 ml of the composition of the present invention. In addition, the active ingredients of the present invention may contain various nutrients, vitamins, minerals (electrolytes), flavoring agents such as synthetic and natural flavoring agents, coloring agents, and enhancers (cheese, chocolate, etc.), pectic acid and salts thereof, alginic acid and salts thereof, organic acid, a protective colloidal thickener, a pH adjusting agent, a stabilizer, a preservative, glycerin, alcohol, a carbonating agent used in a carbonated drink, etc. In addition, the active ingredient of the present invention may contain pulp for preparing natural fruit juice, fruit juice beverages, and vegetable beverages. These ingredients may be used independently or in combination.

As used herein, the term 'feed composition' may mean any natural or artificial diet, one-meal diet, or ingredients of the one-meal diet to be eaten, ingested, and digested by animals or suitable thereto. The type of feed composition is not particularly limited, and may mean a feed composition commonly used in the art. Non-limiting examples of the feed composition may include vegetable feeds, such as grains, root fruits, food processing by-products, algae, fibers, pharmaceutical by-products, oils and fats, starches, meals, or grain by-products; and animal feeds such as proteins, inorganic materials, oils and fats, minerals, single-cell proteins, animal planktons, or foods. These feed compositions may be used alone or in combination of two or more kinds.

Furthermore, according to various embodiments of the present invention, the feed composition may further include excipients, diluents, and additives.

Hereinafter, the present invention will be described in more detail through Examples. However, these Examples are intended to illustrate one or more embodiments, and the scope of the present invention is not limited to these Examples.

### Example 1: Identification of strains that inhibit activity of antibiotic-resistant bacteria

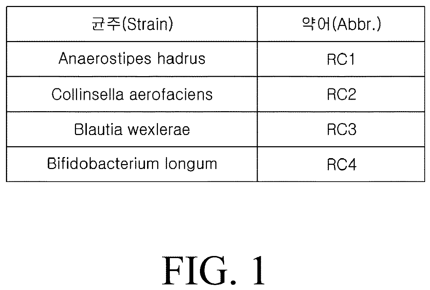

FIG. 1 lists names of strains that inhibit the activity of antibiotic-resistant bacteria included in an antibacterial composition according to an embodiment of the present invention.

According to an embodiment of the present invention, the present inventors obtained human feces suitable for sample selection conditions available for fecal implantation to identify strains that inhibited the activity of antibiotic-resistant bacteria. The feces selected according to the sample selection conditions described above were frozen and stored at -80°C immediately after collection. Strains for inhibiting the activity of antibiotic-resistant bacteria were identified from feces through the following process. (1) About 1 g of feces were dispensed and suspended in 9 ml of sterilized PBS. (2) The suspended supernatant was taken and smeared on a TSMVH medium, cultured in an anaerobic incubator for 48 hours, and then smeared on a solid medium to collect the generated colonies. (3) The gene sequence of the strain identified as a single strain through pure culture was analyzed by 16S rRNA sequencing, and strains with at least 97% homology were identified using NCBI blast.

Meanwhile, the basic safety and physiological activity of all the identified strains described above, such as hemolysis and antibiotic resistance, were verified according to a safety verification method notified by the Ministry of Food and Drug Safety, and whole-genome analysis of each strain was performed to determine the presence or absence of antibiotic resistance genes and other useful genes.

Through this, *Anaerostipes hadrus* (RC1), *Collinsella aerofaciens* (RC2), *Blautia wexlerae* (RC3) and *Bifidobacterium longum* (RC4) strains shown in FIG. 1 were identified as strains that inhibited the activity of antibiotic-resistant bacteria.

Therefore, according to an embodiment of the present invention, the *Anaerostipes hadrus* may be a strain identified by a 16S rDNA base sequence represented by SEQ ID NO: 1, the *Collinsella aerofaciens* may be a strain identified by a 16S rDNA sequence represented by SEQ ID NO: 2, the *Blautia wexlerae* may be a strain identified by a 16S rDNA sequence represented by SEQ ID NO: 3, and the *Bifidobacterium longum* may be a strain identified by a 16S rDNA sequence represented by SEQ ID NO: 4.

### Example 2: Confirmation of VRE inhibitory effect according to combination of strains that inhibit activity of antibiotic-resistant bacteria in synchronous culture

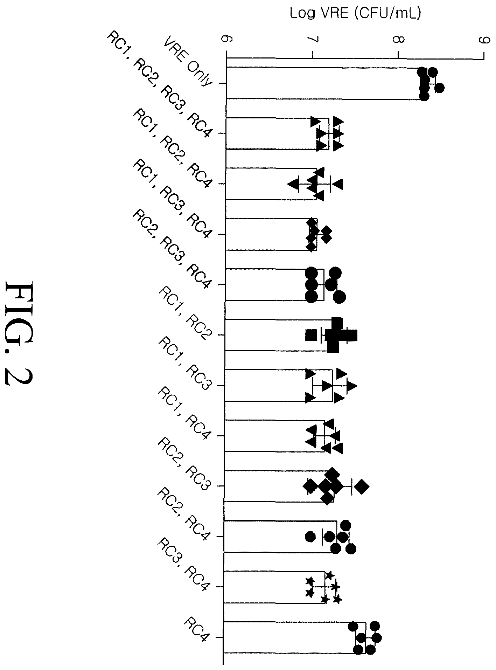

FIG. 2 shows results of effects of inhibiting the activity of Vancomycin-resistant *Enterococci* according to combinations of strains that inhibited the activity of antibiotic-resistant bacteria according to another embodiment of the present invention.

Referring to FIG. 2, according to another embodiment of the present invention, the present inventors intended to verify the activity of inhibiting antibiotic-resistant bacteria according to combinations of strains that inhibited the activity of antibiotic-resistant bacteria and intended to verify an effect of inhibiting the activity of Vancomycin-resistant *Enterococci* (VRE) even among the antibiotic-resistant bacteria.

Specifically, the present inventors intended to compare and verify which combination of one or more strains selected from RC1 to RC4, which were VRE activity-inhibiting strains, exhibited an effect of inhibiting VRE activity.

First, the VRE activity-inhibiting strains RC1 to RC4 were cultured in a TSMVH medium for 18 hours under anaerobic conditions, respectively, the absorbance at 600 nm of each strain culture was measured, and each strain culture was concentrated or diluted so that the absorbance became 1 and then inoculated at 2.5% (v/v) into a newly prepared TSMVH medium, respectively. The combination of RC1 to RC4 strains was inoculated by mixing the mixed cultures of each strain at the same concentration (v/v) and adjusting the total inoculum to 5% (v/v).

The Vancomycin-resistant *Enterococci* (VRE) strain was cultured under anaerobic conditions in a Brain Heart Infusion (BHI) medium for 18 hours and concentrated or diluted so that the absorbance at 600 nm of the VRE strain culture became 1, and then inoculated into a medium inoculated with RC1 to RC4 strains and the combination of strains so that the concentration of the VRE strain culture was 2% (v/v). At this time, the BHI medium was a microbial culture medium commonly used as a microbial selective medium capable of detecting some strains such as antibiotic-resistant bacteria and may refer to a medium made based on extracts extracted from the pig brain and heart.

Thereafter, the combinations of RC1 to RC4 strains and the VRE strain were synchronously cultured under anaerobic culture conditions (95% nitrogen, 5% carbon dioxide) for 16 hours, and then the VRE living cell number in the culture was measured.

Furthermore, one-way ANOVA was performed to compare and analyze a significant difference from a control group (VRE Only), and the results showed a statistical significance in all combinations (****P < 0.0001).

Referring to FIG. 2, FIG. 2 shows the living cell number of VRE per 1 mL of a culture of VRE only or after synchronous culture of VRE and combinations of RC1 to RC4 strains, as the log number, as shown on the vertical axis of FIG. 2. The selected combinations of RC1 to RC4 strains showed slight differences in VRE strain inhibition depending on each composition, but it was confirmed that all combinations of RC1 to RC4 strains and RC4 only inhibited the VRE strain activity. In particular, it was confirmed that the combinations of two or more strains among the R1 to RC4 strains reduced the living cell number of VRE by reduction of the log number of 1 or higher, that is, 1/10 compared to the control group (VRE only).

In particular, it was confirmed that a combination of RC1 to RC4, a combination of RC1, RC2 and RC4, a combination of RC1, RC3 and RC4, and a combination of RC2, RC3 and RC4 had excellent effects.

### Example 3: Confirmation of VRE inhibitory effect according to combination of strains that inhibit activity of antibiotic-resistant bacteria using mouse infectious model

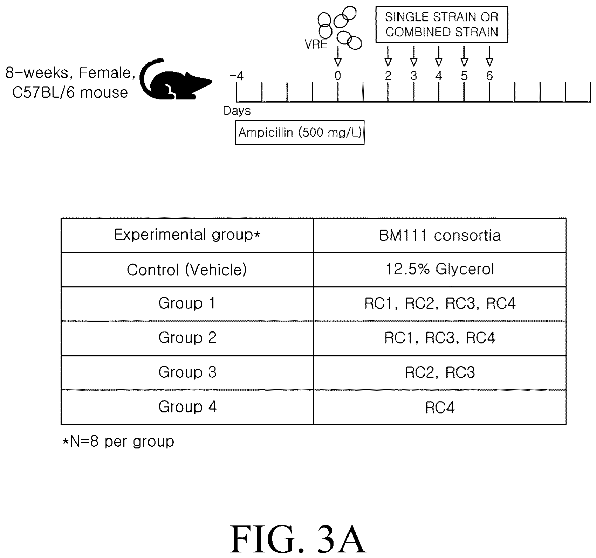

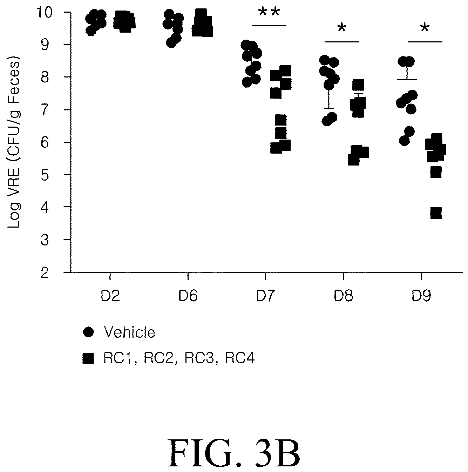

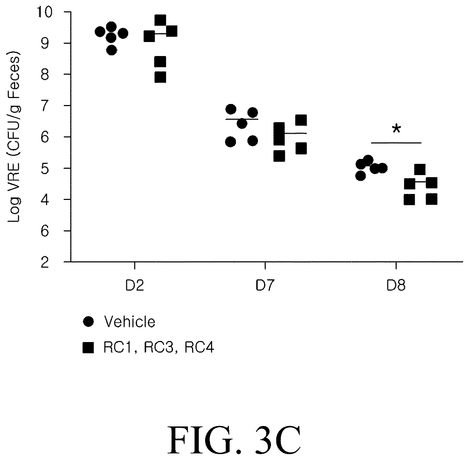

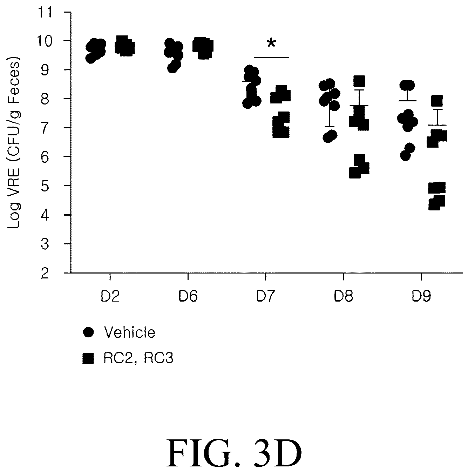

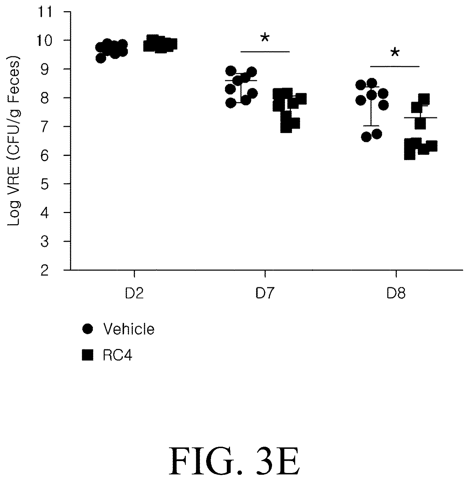

FIG. 3 shows results of inhibiting the activity of Vancomycin-resistant *Enterococci* according to combinations of strains that inhibited the activity of antibiotic-resistant bacteria using a mouse infectious model according to yet another embodiment of the present invention.

Referring to FIG. 3, according to yet another embodiment of the present invention, the present inventors intended to compare and verify using a mouse infectious model which combination of one or more strains selected from RC1 to RC4 as VRE activity-inhibiting strains exhibited the effect of inhibiting VRE activity.

Referring to FIG. 3A, first, drinking water added with ampicillin at a concentration of 500 mg/L was supplied to mice every day for 5 days before infection with the VRE strain. The drinking water added with ampicillin at the concentration of 500 mg/L was supplied to the mice until 1 day after the time of VRE strain inoculation. From day 2 after inoculation with the VRE strain, the drinking water was replaced with antibiotic-free drinking water. The VRE strain inoculum at the time of VRE inoculation (0 days, D0) was 10⁸ CFU, and administered orally in the form of living bacteria. Subsequently, from 2 days (D2) to 6 days (D6) after VRE strain inoculation, the combinations of VRE-inhibiting strains were divided into Group 1 (a combination of RC1, RC2, RC3, and RC4), Group 2 (a combination of RC1, RC3, and RC4), Group 3 (a combination of RC2 and RC3), and Group 4 (RC4 only) and orally administered by 10⁹ CFU/100 mL once a day total 5 times for 5 days. The N number of mice per group was 8. As a control group, 12.5% glycerol was administered orally.

Referring to FIG. 3B, the VRE living cell number per 1 g of mouse feces was counted on the vertical axis at 1 day (D7) and 2 days (D8) after the end time (D6) of oral administration of the VRE-inhibiting strain combinations (Group 1 to Group 4). The VRE living cell number was indicated as the log number as shown on the vertical axis in FIG. 3B.

The VRE living cell number was measured selectively using an *Enterococcal* (EC) agar plate supplemented with 64 ug/ml of vancomycin. A multiple-t test was performed to compare and analyze statistically significant differences from the control group, and the results were expressed as mean ± SD (*P < 0.05).

Referring to FIG. 3B, the number of VREs in D7 of Group 1 was reduced to 6 to 8 as the log number. Furthermore, in D9, Log VRE was reduced to 4 to 6 or less. In contrast, the control group showed a Log VRE distribution of 6 to 9 even in D9. That is, the combinations of VRE-inhibiting strains RC1, RC2, RC3, and RC4 showed the ability of inhibiting VRE activity 100 times greater than the control group within 2 days after oral administration.

Furthermore, even in D9 of Group 3, which is a combination of VRE-inhibiting strains of RC2 and RC4, Log VRE showed a decrease in the VRE number up to 4. On the other hand, the control group showed a Log VRE distribution of 6 to 8 even in D9.

Group 2, a combination of RC1, RC3, and RC4 strains, also showed the VRE inhibition ability compared to the control group. However, unlike an *in vitro* experiment of Example 2, Group 2 showed slightly lower VRE inhibition ability than Group 1 or Group 3. Likewise, unlike the *in vitro* experiment of Example 2, the RC4-only strain of Group 4 also showed slightly lower VRE inhibition ability than Group 1 or Group 3.

Furthermore, in experiments for comparing the effects of inhibiting the VRE activity using a mouse infectious model according to various embodiments of the present invention, each strain only of the strains RC1 to RC4 did not show improved VRE inhibition ability compared to the control group (not shown).

When summarizing the results of Example 3 with reference to the results of Example 2, it was confirmed that combinations of two or more strains among the RC1 to RC4 strains showed further improved VRE inhibitory activity compared to the control group even in a mouse animal experimental model.

### <Preparation and administration of VRE strain>

A VRE strain inoculated into mice was cultured and prepared through the following process. First, the present inventors took a single colony of the VRE strain from a pure cultured EC agar plate, inoculated the single colony into a fresh LB liquid medium, cultured the colony for about 24 hours, and then inoculated the colony at a concentration of 1% (v/v) in a fresh Lysogeny broth (LB) medium. Subsequently, the present inventors measured the absorbance after shaking-culturing for 4 hours at 37°C and 180 rpm and concentrated or diluted to be the living cell number of 10⁸ CFU per 100 µL. 10⁸ CFU per dose was orally administered to each mouse.

### <Preparation and administration of VRE-inhibiting strain>

The present inventors inoculated 2.5% of VRE-inhibiting strains, that is, strains in Group 1 to Group 4, into a TSMVH medium, cultured the strains in an anaerobic chamber (95% nitrogen, 5% carbon dioxide) for 24 hours, and then concentrated or diluted the cultured strains to become the absorbance of 1. Next, each group was concentrated or diluted in a phosphate buffer solution to be finally 10⁹ CFU/100 µL, and 100 µL was orally administered to mice in each group. In a control group, 100 µL of 12.5% glycerol was administered orally.

### Example 4: Comparison of VRE inhibitory effect using mouse infectious model

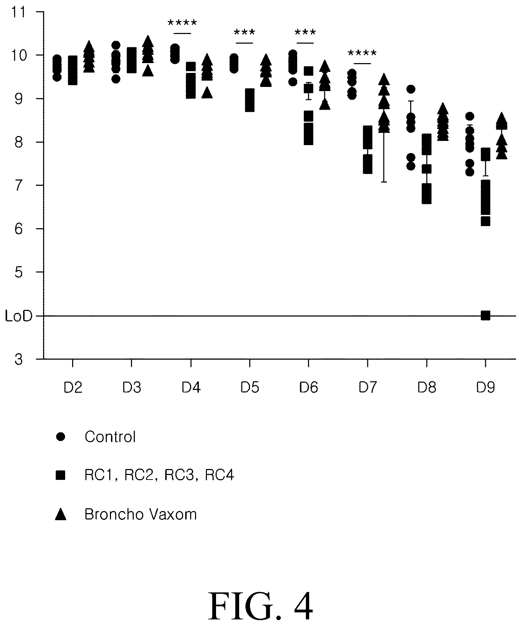

FIG. 4 shows comparison of effects of inhibiting (suppressing) VRE on combinations (hereinafter, combinations of VRE-inhibiting strains) of four strains RC1, RC2, RC3, and RC4 according to various embodiments of the present invention and Bronco Vaxom (BV), currently available as an infection prevention agent, as a comparative group.

Referring to FIG. 4, first, drinking water added with 500 mg/L of ampicillin was supplied to mice every day for 5 days before infection with the VRE strain. The drinking water added with 500 mg/L of ampicillin was supplied to the mice until 1 day after the time of VRE strain inoculation.

From day 2 after inoculation with the VRE strain, the drinking water was replaced with antibiotic-free drinking water. The VRE strain inoculum at the time of VRE inoculation (0 days, D0) was 10⁸ CFU, and administered orally in the form of living bacteria. Subsequently, from 2 days (D2) to 6 days (D6) after VRE strain inoculation, combinations of VRE-inhibiting strains were orally administered by 10⁹ CFU/100 µL once a day total 5 times for 5 days. As a control group, 12.5% glycerol was administered orally. In a comparative group (Bronco Vaxom), 2 mg per mouse was administered.

Referring to FIG. 4, from 2 days (D2) to 9 days (D9) after inoculation with the VRE strain, mouse feces were taken every day and the VRE living cell number was measured, which was indicated as the log number of the VRE living cell number per 1 g of feces as shown on the vertical axis in FIG. 6. The VRE living cell number was measured selectively using an *Enterococcal* (EC) agar plate supplemented with 64 µg/ml of vancomycin. A multiple-t test was performed to compare and analyze statistically significant differences between the control group and the comparative group, and the results were expressed as mean ± SD (***P < 0.005; ****P < 0.0001).

Referring to FIG. 4, the combinations of VRE-inhibiting strains according to various embodiments of the present invention showed a more improved VRE inhibitory effect than the control group or the comparative group.

Specifically, the combinations of VRE-inhibiting strains showed a statistically significant VRE inhibitory effect from D4, and this inhibitory effect was further increased up to D9.

That is, it was confirmed that a combination (BM111) of RC1, RC2, RC3, and RC4 strains showed more improved VRE inhibition activity than the control group or the comparative group (Bronco Vaxom, BV).

### Example 5: Comparison of effects of inhibiting antibiotic-resistant bacteria using mouse infectious model

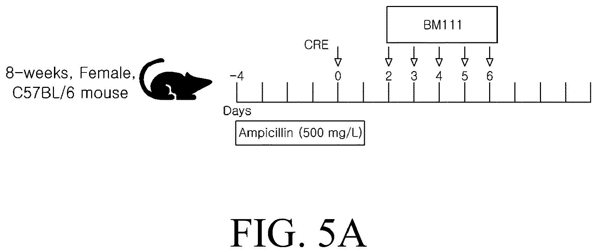

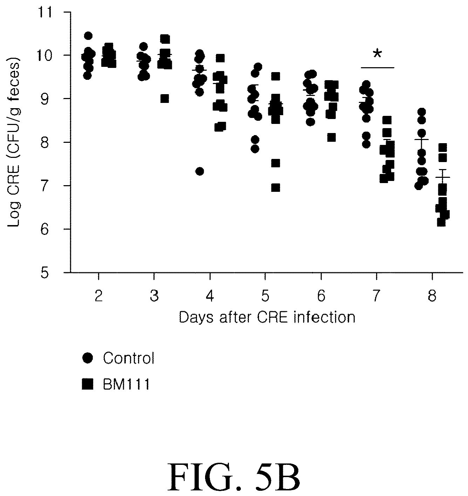

FIGS. 5A and 5B show results of inhibiting the activity of carbapenem-resistant *Enterobacteriaceae* (CRE) according to administration of a combination (hereinafter, BM111) of strains RC1, RC2, RC3, and RC4 which is a combination of stains which inhibit the activity of antibiotic-resistant bacteria of the present invention in a mouse infectious model according to various embodiments of the present invention.

Referring first to FIG. 5A, in order to confirm whether combinations of mixed strains of the present invention may effectively inhibit CRE, the efficacy of each combination was verified using a mouse infectious model. From 5 days before CRE infection, drinking water added with ampicillin at a concentration of 500 mg/L was supplied, replaced daily, supplied until the next day after CRE inoculation, and then replaced with antibiotic-free drinking water. On day 5 after CRE inoculation, each mouse was orally administered and infected with 10⁹ CFU of a Carbapenem-resistant *E. coli* strain isolated from a patient showing infection. For the CRE strain, a single colony was taken from a pure cultured Tryptic soy broth (TSB) agar medium, inoculated into a fresh Brain Heart Infusion (BHI) liquid medium, cultured for 1 day, and then inoculated at 1% into a new BHI liquid medium the next day. The single colony was cultured for 4 hours at 37°C and 180 rpm conditions, the living cell number was adjusted by measuring OD. 10⁸ CFU was orally administered to each mouse, and from 1 day after CRE infection, an experimental strain was orally administered at the same concentration every day for 5 days. The experimental strain was prepared in an anaerobic chamber (95% N₂, 5% CO₂), inoculated at 2.5% into a new TSB + Vitamin K1, L-cysteine medium, cultured for 24 hours, and then adjusted to OD = 1. The prepared strain solution was adjusted so that the final concentration of each strain was adjusted to 10⁹ CFU/100 µl of PBS, and 100 µl of each strain was orally administered to each mouse. In a control group, 100 µl of 12.5% glycerol was administered orally, and feces were collected and counted every day after oral administration of the strain solution was completed. The CRE living cell number was measured by selecting and counting the corresponding colonies after culturing in ChromaCRE media at 37°C for 24 hours.

Subsequently, referring to FIG. 5B, FIG. 5B is a diagram showing the results of counting a change in the number of Carbapenem-resistant *E. coli* strains, and it was confirmed that a group treated with BM111 showed a significant effect from day 5 compared to the control group.

### Example 6: Comparison of effects of inhibiting antibiotic-resistant bacteria using mouse infectious model

FIGS. 6A to 6C show results of inhibiting the activity of *Klebsiella pneumoniae* as Carbapenem-resistant *Enterobacteriaceae* (CRE) according to administration of a combination (hereinafter, BM111) of strains RC1, RC2, RC3, and RC4 which is a combination of stains which inhibit the activity of antibiotic-resistant bacteria of the present invention in a mouse infectious model according to various embodiments of the present invention.

First, referring to FIG. 6A, in order to confirm the effect of inhibiting antibiotic-resistant bacteria using a mouse infectious model, a humanized mouse infectious model was established by implanting feces from a patient with CRE/VRE infection and used to measure the efficacy of BM111. Specifically, the feces of the patients infected with CRE/VRE and scheduled for treatment were analyzed, and the feces of a total of six patients showing a high level of infection with 1% or more of CRE and VRE present in the feces were collected and homogenized into one sample. About 8 mg of the homogenized feces was orally administered to each mouse. The degree of intestinal VRE/CRE infection was determined 2 days later in mice implanted with feces of the CRE/VRE infected patients, and a total cell number of 1 × 10⁹ CFU was orally administered total 5 times every 24 hours by adjusting the living cell number of each strain constituting BM111 to be 2.5 × 10⁸ CFU per mouse. After oral administration, mouse feces were collected for each hour, and the number of intestinal bacteria was measured for CRE using a CHORM mSuperCarba agar medium (Asan Pharmaceutical, AM945-01).

As a result, subsequently, referring to FIG. 6B, it was confirmed that a change in the number of Carbapenem-resistant *Escherichia coli* in FIG. 6B(a)showed a significant change from day 4 after treatment with BM111. It was confirmed that a change in the number of Carbapenem-resistant *K. pneumonia* in FIG. 6B(b) showed a significant change from day 4 after treatment with BM111, and thus, it was confirmed that the BM111 strain combination of the present invention was effective in inhibiting the activity of Carbapenem-resistant bacteria.

Subsequently, referring to FIG. 6C, FIG. 6C is a diagram illustrating results of confirming a CRE strain inhibitory effect according to a strain combination in a synchronous culture. Specifically, in order to compare and verify the CRE inhibition effects of combinations of 2, 3, and 4 strains, O.D. was measured respectively for strains cultured in TSV (non-animal TSB + L-cysteine, Vitamin K1) under anaerobic conditions for 18 hours, and adjusted to O.D₆₀₀ₙₘ = 1, and then in the case of mixed strains, the concentration of each strain was mixed at the same concentration and adjusted so that the total inoculum became 5%. In the case of CRE, Carbapenem-resistant *K. pneumonia* isolated from infected patients was cultured in a BHI medium for 18 hours and then adjusted to OD = 1 and inoculated at final 2% by each 1% into a medium inoculated with a candidate strain. After synchronous culture under anaerobic conditions for 16 hours, the living cell number of Carbapenem-resistant *K*. *pneumonia* was measured. The living cell number of Carbapenem-resistant *K. pneumonia* was measured by selecting and counting using *Enterococcal* (EC) media added with 64 ug/ml of vancomycin. FIG. 6C shows counting of the living cell number of *K. pneumonia* under culture conditions, and it was confirmed that the combination of selected strains showed a slight difference in the degree of strain inhibition depending on each composition, but in a specific combination, the growth of Carbapenem-resistant *K. pneumonia* was inhibited. At this time, as a result of performing one-way ANOVA to compare and analyze a significant difference from the control group, and selecting cases (*P < 0.05, **P < 0.01, ***P < 0.001) with a significant difference, it was confirmed that in a combination including all RC1 to RC4, a combination including RC1 to RC3, a combination including RC1, RC2 and RC4, a combination including RC1, RC3 and RC4, a combination including RC2 and RC4, and a combination including RC3 and RC4 among RC1 to RC4 of the present invention, an effect of inhibiting Carbapenem-resistant *K. pneumonia* was excellent. Therefore, it was confirmed that there was an excellent effect of inhibiting the activity of antibiotic-resistant bacteria by using a composition of the present invention including two or more strains selected from the group consisting of *Anaerostipes hadrus, Collinsella aerofaciens, Blautia wexlerae,* and *Bifidobacterium longum* of the present invention.

Although the embodiments of the present invention have been described in detail with reference to the accompanying drawings, the present invention is not limited thereto and may be embodied in many different forms without departing from the technical concept of the present invention. Therefore, the embodiments of the present invention are provided for illustrative purposes only but are not intended to limit the technical concept of the present invention. The scope of the technical concept of the present invention is not limited thereto. Therefore, it should be appreciated that the aforementioned embodiments are illustrative in all aspects and are not restricted. The protective scope of the present invention should be construed on the basis of the appended claims, and all the technical ideas in the equivalent scope thereof should be construed as falling within the scope of the present invention.

## Claims

1. An antibacterial composition comprising two or more strains selected from the group consisting of *Anaerostipes hadrus, Collinsella aerofaciens, Blautia wexlerae* and *Bifidobacterium longum* strains.

2. The antibacterial composition of claim 1, wherein the *Anaerostipes hadrus, Collinsella aerofaciens, Blautia wexlerae* and *Bifidobacterium longum* strains are isolated from human feces.

3. The antibacterial composition of claim 1, wherein the *Anaerostipes hadrus* is *Anaerostipes hadrus* (accession number: KCTC15409BP),
the *Collinsella aerofaciens* is *Collinsella aerofaciens* (accession number: KCTC15408BP),
the *Blautia wexlerae* is*Blautia wexlerae* (accession number: KCTC15396BP), and
the *Bifidobacterium longum* is *Bifidobacterium longum* (accession number: KCTC15416BP).

4. The antibacterial composition of claim 3, wherein the composition includes the *Anaerostipes hadrus* (accession number: KCTC15409BP), *Collinsella aerofaciens* (accession number: KCTC15408BP), and *Blautia wexlerae* (accession number: KCTC15396BP).

5. The antibacterial composition of claim 3, wherein the composition includes the *Anaerostipes hadrus* (accession number: KCTC15409BP), *Blautia wexlerae* (accession number: KCTC15396BP), and *Bifidobacterium longum* (accession number: KCTC15416BP).

6. The antibacterial composition of claim 3, wherein the composition includes the *Collinsella aerofaciens* (accession number: KCTC15408BP), *Blautia wexlerae* (accession number: KCTC15396BP), and *Bifidobacterium longum* (accession number: KCTC15416BP).

7. The antibacterial composition of claim 1, wherein the *Anaerostipes hadrus* is a strain identified by a 16S rDNA base sequence represented by SEQ ID NO: 1,
the *Collinsella aerofaciens* is a strain identified by a 16S rDNA sequence represented by SEQ ID NO: 2,
the *Blautia wexlerae* is a strain identified by a 16S rDNA sequence represented by SEQ ID NO: 3, and
the *Bifidobacterium longum* is a strain identified by a 16S rDNA sequence represented by SEQ ID NO: 4.

8. The antibacterial composition of claim 1, wherein the strains have antibiotic-resistant bacteria inhibitory activity.

9. The antibacterial composition of claim 8, wherein the antibiotic-resistant bacteria are Vancomycin-resistant *Enterococci.*

10. A pharmaceutical composition for preventing or treating Vancomycin-resistant *Enterococci* (VRE) infections, comprising as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit activity of the Vancomycin-resistant *Enterococci,* a culture of the strain, a concentrate of the culture, a dried product of the culture, and combinations thereof.

11. The pharmaceutical composition for preventing or treating VRE infections of claim 10, wherein the *Anaerostipes hadrus, Collinsella aerofaciens, Blautia wexlerae,* and *Bifidobacterium longum* strains are administered by one or more routes selected from the group consisting of oral, rectal, and colonic routes.

12. A health functional food for inhibiting activity of Vancomycin-resistant *Enterococci,* comprising as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit the activity of Vancomycin-resistant *Enterococci,* a culture of the strain, a concentrate of the culture, a dried product of the culture, and combinations thereof.

13. A feed additive for inhibiting activity of Vancomycin-resistant *Enterococci,* comprising as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit the activity of Vancomycin-resistant *Enterococci,* a culture of the strain, a concentrate of the culture, a dried product of the culture, and combinations thereof.

14. A method for treating Vancomycin-resistant *Enterococci* (VRE) infections in animals other than humans, comprising administering the composition of claim 10 to a subject other than a human suspected of having the Vancomycin-resistant *Enterococci* (VRE) infections.

15. The antibacterial composition of claim 8, wherein the antibiotic-resistant bacteria are Carbapenem-resistant *Enterobacteriaceae* (CRE).

16. A pharmaceutical composition for preventing or treating Carbapenem-resistant *Enterobacteriaceae* (CRE) infections, comprising as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit activity of the Carbapenem-resistant *Enterobacteriaceae* (CRE), a culture of the strain, a concentrate of the culture, a dried product of the culture, and combinations thereof.

17. The pharmaceutical composition for preventing or treating CRE infections of claim 16, wherein the *Anaerostipes hadrus, Collinsella aerofaciens, Blautia wexlerae,* and *Bifidobacterium longum* strains are administered by one or more routes selected from the group consisting of oral, rectal, and colonic routes.

18. A health functional food for inhibiting activity of Carbapenem-resistant *Enterobacteriaceae* (CRE), comprising as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit the activity of Carbapenem-resistant *Enterobacteriaceae* (CRE), a culture of the strain, a concentrate of the culture, a dried product of the culture, and combinations thereof.

19. A feed additive for inhibiting activity of Carbapenem-resistant *Enterobacteriaceae* (CRE), comprising as an active ingredient at least one selected from the group consisting of two or more strains selected from the group consisting of *Anaerostipes hadrus, Collinsella aerofaciens, Blautia wexlerae,* and *Bifidobacterium longum* strains, which inhibit the activity of Carbapenem-resistant *Enterobacteriaceae* (CRE), a culture of the strain, a concentrate of the culture, a dried product of the culture, and combinations thereof.

20. A method for treating Carbapenem-resistant *Enterobacteriaceae* (CRE) infections in animals other than humans, comprising administering the composition of claim 16 to a subject other than a human suspected of having the Carbapenem-resistant *Enterobacteriaceae* (CRE) infections.
